# EUROPEAN PATENT APPLICATION

(11) **EP 1 438 969 A1**
(43) Date of publication of application: **21.07.2004**
(21) Application number: 03075117.6
(22) Date of filing: 14.01.2003
(51) Int. Cl.: A61K 39/12, C07K 14/08, G01N 33/569, C07K 16/10

(54) **Arterivirus marker vaccine**

(71) Applicant: Universiteit Utrecht, 3584 CS Utrecht (NL)
(72) Inventor: The designation of the inventor has not yet been filed
(74) Representative: Prins, Adrianus Willem, Mr. Ir.

(57) **Abstract**

The invention relates to arterivirus compositions used as vaccines to induce protective immunity against infection or disease caused by arteriviruses. Provided is a vaccine directed against infection or disease caused by an arterivirus, said vaccine comprising an arterivirus provided with a modification in a nucleic acid sequence allowing to distinguish animals vaccinated with said vaccine from animals infected with wild-type arterivirus or vaccinated with an unmodified arterivirus strain. Also provided is a method for distinguishing unvaccinated animals or animals vaccinated with a vaccine according to the invention from animals infected with wild-type arterivirus or vaccinated with an unmodified arterivirus strain.

## Description

The invention relates to arterivirus compositions used as vaccines to induce protective immunity against infection or disease caused by arteriviruses. Arteriviruses represent a family of viruses within the order *Nidovirales.* Presently known members of the family are Equine Arteritis Virus (EAV), Porcine Reproductive and Respiratory Syndrome virus (PRRSV), Simian Hemorrhagic Fever Virus (SHFV) and Lactate Dehydrogenase-elevating Virus (LDV). (For reviews, see Plagemann and Moennig, 1998; Glaser et al., 1997; de Vries et al., 1997; Snijder and Meulenberg, 1998; Dea et al., 2000). These are enveloped, plus-stranded RNA viruses. Their genome consists of a (approximately 13 to 16 kb long) 5'-capped and 3'-polyadenylated RNA molecule that is packaged by nucleocapsid (N) molecules into a capsid which is in turn surrounded by a lipid membrane or envelope to yield a single, fully assembled virus particle, also known as a virion. Within this envelope 6 membrane proteins occur. The predominant ones are an unglycosylated protein M and a glycoprotein that is variably called G_{L} (EAV), GP5 (PRRSV, SHFV) or VP-3P (LDV). These two proteins occur in virions as disulfide-linked heterodimers. In addition to these major envelope proteins, one unglycosylated protein - the E protein - is found with an intermediate abundance in the membrane of these viruses while 3 minor glycoproteins have been identified named GP4, GP3 and GP2 (or Gs in EAV).

Little is known about the functions of the envelope proteins. It is generally assumed that the two major envelope proteins play an essential role in the intracellular assembly of these viruses by interacting with the nucleocapsid during the formation and budding of the viral particles. These proteins, by the structures that the heterodimers constitute on the outside surface of virions, are also supposed to be instrumental during entry of the host cell. However, a role in specific targeting, i.e. in directing the virus to the host cell receptor, has become unlikely recently by the demonstration, using infectious cDNA clones, that the exchange of the ectodomain of the EAV G_{L} protein and of the PRRSV M protein by ectodomains of corresponding proteins from other arteriviruses did not alter the chimeric viruses' tropism. Though this result does not rule out another function of the heterodimers in cell entry, it suggests that the minor proteins might be involved in receptor targeting.

Arteriviruses each have quite a narrow host range. EAV infects horses and donkeys (equids), LDV is strictly mouse-specific, PRRSV infects pigs while SHFV infection is restricted to (certain genera of) monkeys. Arteriviruses are easily spread by the respiratory route, through direct contact between animals, via aerosols as well as by virus secreted with urine and faeces. Another important route of transmission is by semen, the virus being secreted with the ejaculate by acutely or persistently infected males.

Macrophages are a major target cell for all arteriviruses in their natural host. The outcome of an infection may vary greatly depending on many conditions such as age and health status of the animal, the strain of the infecting virus and the route of infection. Thus, infection with EAV and PRRSV may result in a wide spectrum of disease, from being asymptomatic or causing only mild symptoms (respiratory problems, transient fever, necrosis of small muscular arteries), to affecting the animals severely. Reproductive failure is also a common clinical outcome, often characterized by abortion of the foetus by the pregnant mares or sows. Altogether these infections cause great economical losses.

Another common feature of arteriviruses is their ability to cause persistent infection, which for EAV and PRRSV is restricted to male animals. In stallions and boars the infection can apparently not be cleared effectively from the reproductive organs by the immune system. As a result, virus continues to be secreted with the semen, which constitutes an important factor in the epidemiology of these infections.

While little is known about the cellular immune responses during arterivirus infection, analyses of humoral responses have generally shown the appearance of antibodies against all the structural proteins. The G_{L} /GP5 protein is the major antigen against which virus-neutralizing antibodies are elicited. For PRRSV, neutralizing epitopes are apparently also associated with GP4 and GP3 as was shown using monoclonal antibodies and baculovirus expression products, respectively, although the results have not always been consistent and their overall contribution to virus neutralization is considered limited.

Both live attenuated and killed virus vaccines are commercially available for the two economically important arteriviruses, EAV and PRRSV. The efficacy of live vaccines is generally higher and the immunity lasts longer as compared to inactivated virus vaccines. Two commercial tissue culture vaccines have been produced against EAV. One is a modified live virus vaccine prepared from virus that has been attenuated for horses by multiple serial transfers in equine and rabbit cell cultures. It has been shown to be safe and protective for stallions and nonpregnant mares. Vaccination of foals under 6 weeks of age and of pregnant mares in the final 2 months of gestation is contraindicated. The second vaccine is an inactivated, adjuvanted product prepared from virus grown in equine cell culture that can be used in nonbreeding and breeding horses. In the absence of appropriate safety data, the vaccine is not currently recommended for use in pregnant mares.

For arteriviruses, such as PRRSV, the genetic diversity - clearly different viruses have appeared to circulate in Europe and North America - poses serious problems for the development of vaccines that provide broad protection against these different variants.

Despite their better performance, live vaccines still need to be improved because the current vaccine viruses can spread and do not protect against re-infection. Importantly, current vaccine viruses cannot be discriminated from field viruses. For the latter reason the use of live or killed arterivirus vaccines is restricted in some countries (e.g. in parts of the US and in the UK) as this might undermine the maintenance of effective serological monitoring required for the early control of outbreaks.

Various alternative vaccine candidates have been tested, most of them aimed at the induction of protective antibodies against the major glycoprotein G_{L} /GP5. For EAV, for instance, bacterial expression products comprising the entire G_{L} ectodomain or part thereof, and a synthetic peptide derived from it each induced virus neutralizing antibodies in horses which, when tested in the case of the complete G_{L} ectodomain subunit vaccine, appeared to provide protection against challenge with virulent virus. Similarly protective in horses was a recombinant alphavirus co-expressing the two major EAV envelope proteins G_{L} and M. Another approach to induce protective immunity based on GP5 is vaccination with DNA expressing PRRSV ORF5. The induction of neutralizing antibodies as well as GP5-specific lymphocytes in pigs was observed which protected the animals markedly against challenge with a virulent strain of the virus. Studies with neutralization-resistant EAV mutants generated by growing viruses in the presence of neutralizing monoclonal antibodies revealed that the membrane-proximal region of the G_{L} ectodomain is the immunodominant region (Balasuriya et al., 1997). Two monoclonal escape mutant viruses were found to have deletions of residues 62-101 and 66-112, respectively. Neutralization of these viruses by immune sera from EAV-infected horses was significantly though not completely reduced, while an anti-EAV hyperimmune serum raised in rabbits failed to neutralize both viruses completely.

A variety of diagnostic assays has been described for the detection of EAV and PRRSV. For EAV the classical test is virus isolation in culture cells but in addition many serological tests have been described including virus neutralization and various ELISA tests. The latter have been based particularly on whole virus and on the G_{L}, M and N proteins. The latter tests made use of differently expressed proteins, either expressed entirely or partly, and used as such or fused or conjugated to a foreign protein. Also, synthetic peptides derived from the major structural proteins have been applied. ELISA tests based on the immunodominant G_{L} protein ectodomain generally perform very well as we demonstrated recently (Nugent et al., 2000).

As illustrated by these observations there is an overwhelming body of evidence that immunity against these viruses is mediated by neutralizing antibodies, particularly by antibodies directed against the major surface glycoprotein. This is certainly the case for EAV where, for instance, the appearance of neutralizing antibodies in the serum of infected horses coincides with the elimination of virus from the circulation and where the passive transfer of maternal antibodies through the colostrum of immune mares reduces or prevents viral arteritis in foals. Also for PRRSV data indicate a protective role of neutralizing antibodies present in colostrum transferred to piglets. Likewise, neutralizing antibodies passively transferred to pigs cleared PRRSV viraemia effectively; such transferred antibodies also prevented transplacental infection and completely cleared PRRSV infection in pregnant sows. Seroneutralization of PRRSV correlates with the antibody response to the GP5 glycoprotein, not with that to the GP3 and GP4 glycoproteins.

Except for EAV, the neutralizing epitopes present in the major arterivirus envelope glycoprotein are poorly characterized. A number of studies using monoclonal antibodies have elucidated the immunologically important domains in the EAV G_{L} protein. These are all localized in the hydrophilic, exposed part of the glycoprotein constituted by its approximately 115 N-terminal residues. Four distinct neutralization sites were defined including amino acids 49 (site A), 61 (site B), 67 through 90 (site C) and 99 through 106 (site D). With the exception of site A, all these sites are located in the membrane-proximal variable region V1 that comprises amino acids 61-121. These sites are also compatible with the finding that neutralizing antibodies were elicited in horses after immunization with a bacterial fusion protein containing residues 55 through 98 as well as with a synthetic peptide composed of residues 75-97. Similarly, a peptide comprising amino acids 93-112 induced neutralizing antibodies in mice.

Thus, the available data indicate that envelope proteins of arteriviruses are highly immunogenic. After infection, envelope proteins can elicit a strong neutralizing antibody response. Induction of such an antibody response is a critical factor for a successful vaccine. However, an antibody response against wild-type virus strains cannot be distinguished from an antibody response against virulent field virus strains. As a consequence, infections with virulent field strains cannot be detected by serological methods. This situation is undesirable since field virus infections are masked by vaccination. Also, clinical signs that are caused by field strains may be overlooked. Taken together, there is a clear need for a safe arterivirus 'marker' vaccine which will be suitable for widespread use, without compromising disease surveillance via serological screening.

The present invention provides the insight that a recombinant arterivirus lacking a major part (47 of the 97 residues) of the ectodomain of a major envelope protein is infectious to animals, elicits negligible symptoms, yet protects these animals against disease after challenge with a virulent strain of the virus. Provided is a vaccine directed against infection or disease caused by an arterivirus, said vaccine comprising an arterivirus provided with a modification in a nucleic acid sequence encoding a viral protein allowing to distinguish animals vaccinated with said vaccine from animals infected with wild-type arterivirus or vaccinated with an unmodified arterivirus strain or strain modified in other than as provided herein.
A modification comprises at least deletion of at least one nucleic acid or substitution of at least one nucleic acid or addition of at least one nucleic acid. Such nucleic acid modifications or mutations often result in the production of a viral protein or polypeptide with an altered amino acid sequence.

A vaccine according to the invention capable of inducing protective immunity in an animal comprises an arterivirus wherein the amino acid sequence of a viral protein or a viral polypeptide is modified. A viral protein can be a glycoprotein as well as an unglycosylated protein. For example, it is an envelope protein or a core protein. Successful differentiation between vaccination and infection is essential for the eradication of EAV and other arterivirus-mediated disease. By virtue of the modification, an arterivirus according to the invention can be used as a marker vaccine virus because a serological distinction can be made between animals that have been infected with wild-type virus and animals vaccinated with a vaccine according to the invention.

The invention provides a vaccine wherein for example the nucleic acid sequence encoding an ectodomain of a viral envelope protein is modified. Such a modification can be the deletion of one or more amino acids in the ectodomain, for example in the membrane-proximal region, of an envelope protein. Also, a modification comprises substitution of one or more amino acid residues of a viral protein. An amino acid substitution can be a conservative or a nonconservative substitution. Furthermore, stretches of a few amino acids can be substituted or replaced by other stretches of amino acids.

Provided is a vaccine directed against infection or disease caused by an Equine Arteritis Virus (EAV), said vaccine comprising an EAV strain provided with a modification in a nucleic acid sequence encoding a viral protein allowing to distinguish animals vaccinated with said vaccine from animals infected with wild-type EAV or vaccinated with an unmodified EAV strain. For example, a vaccine comprises an EAV wherein an envelope protein is modified. As is exemplified in the detailed description, the invention provides a vaccine comprising a mutant arterivirus lacking part of the G_{L} protein. Whereas it has been suggested that the membrane-proximal immunodominant region of the G_{L} protein ectodomain is essential for the induction of protective immunity, the inventors unexpectedly observed that a recombinant EAV lacking the nucleic acid sequence encoding the amino acid residues 66 to 112 of said G_{L} protein is still infectious to horses, elicits negligible symptoms, but protects these animals against disease after challenge with a virulent EAV strain. This is quite contrary to earlier findings that neutralizing antibodies were elicited in horses after immunization with a bacterial fusion protein containing residues 55 through 98 as well as with a synthetic peptide composed of residues 75-97, as well as that a peptide comprising amino acids 93-112 induced neutralizing antibodies in mice. Thus, the invention provides a vaccine directed against infection or disease caused by an arterivirus, said vaccine comprising an arterivirus provided with a modification in a nucleic acid sequence, wherein said modification preferably comprises the deletion of the nucleic acid sequence encoding the amino acid residues 66 to 112 of said G_{L} protein. A suitable vaccine according to the invention however also comprises an arterivirus wherein a larger or a smaller stretch or segment of an envelope protein, such as the G_{L} protein, is modified, preferably at a region corresponding to approximately the region of amino acid residues 66 to 112 of the EAV G_{L} protein. For example, a stretch showing at least partial overlap with said region is deleted to yield an arterivirus vaccine allowing to distinguish animals vaccinated with said vaccine from animals infected with wild-type arterivirus. Preferably, said deletion renders the virus viable. Such a deleted stretch has for instance a length of around 40 amino acids, or it can be smaller than that like between 30 and 35 residues or even smaller such as 25 to 30 residues. Viability of a virus can be determined using various procedures which are known to a person skilled in the art.

Thus, by virtue of the deletion of a region of contiguous amino acids of G_{L}, which is an important site for development of neutralizing antibodies, a virus is obtained that is still sufficiently immunogenic to provide protection against disease following subsequent EAV challenge infection. Such a virus is furthermore advantageously used as a marker vaccine virus because a serological distinction can be made between animals that have been infected with wild-type virus and animals vaccinated with the mutant virus. It is also possible to replace or exchange one or more domains of a viral protein by one or more domains of a protein from another organism (including a virus) to form a chimeric viral protein, provided that such a chimeric protein allows induction of an effective immune response and serological distinction between mutant and wild-type virus.

It is important to recognize that the present invention is not limited to providing a marker vaccine for EAV. On the contrary, it also provides a useful marker vaccine for disease or infection caused by other arteriviruses, including Porcine Reproductive and Respiratory Syndrome Virus (PRRSV), Simian Hemorrhagic Fever Virus (SHFV), Lactate Dehydrogenase-elevating Virus (LDV) and the like. For example, the GP5 envelope protein of PRRSV or SHFV can be modified according to the invention. Two epitopes were recently mapped in the GP5 ectodomain of PRRSV: a neutralizing epitope (epitope B; amino acids 37-45) which is highly conserved between arteriviruses, and a non-neutralising epitope (epitope A; amino acids 27-30). In one embodiment of the invention, an envelope protein of an arterivirus is modified at a region corresponding to the A and/or B epitope of PRRSV GP5, to yield a vaccine that can induce an effective immune response against the infectious agent and allowing scrological distinction between an animal infected or vaccinated with wild-type PRRSV and animals vaccinated with said modified virus. In another embodiment, the nucleic acid sequence of an arterivirus encoding an ectodomain of an envelope protein is partially or fully replaced by a nucleic acid sequence derived from a different arterivirus, for example a sequence encoding at least part of an ectodomain of an envelope of said different arterivirus. In yet another embodiment of the invention, at least one amino acid residue in a protein of LDV is changed or deleted, such as the VP-3P protein, such that the vaccine is capable of inducing protective immunity against LDV yet allowing differentiation between LDV-infected and LDV-vaccinated animals.

The invention provides a live vaccine or a killed (inactivated) vaccine comprising an arterivirus provided with a modification in a nucleic acid sequence encoding a viral protein. Preferably, such a vaccine is a live vaccine. Induction of appropriate immunity depends on the properties of the antigen. Live vaccines are likely to contain the greatest number of antigens. Living vaccines have great advantage of providing an increased antigenic challenge that lasts days or weeks, and inducing it at the right site-which in practice is most important where mucosal immunity is concerned. In a preferred embodiment, a live but avirulent or attenuated vaccine is provided wherein the virulence of a live virus is diminished while retaining the desired antigens. Several methods can be used to generate an attenuated organism. The most common way to achieve attenuation is by growing a virulent organism in culture or other modified conditions until the strain becomes avirulent. Alternatively, the virulence genes of an infectious agent can be removed through genetic engineering. Furthermore, heterologous strains that are virulent in one species but not virulent in another also have the potential to be used as live vaccines.

However, it should be noted that killed vaccines as provided by the invention are beneficial under those circumstances where a live vaccine is not or only little applicable, for example because of trade restrictions or other conditions set by disease controlling authorities.

Provided is a composition at least comprising an effective amount of vaccine according to the invention which lowers the average clinical symptoms of a group of animals inoculated with said composition, then subsequently challenged with virulent arterivirus, relative to a group of identically challenged animals not inoculated with the composition. As is described in the detailed description, the invention for example provides a vaccine comprising a mutant EAV (EAV-G_{L}Δ). Immunisation with a virus inoculum resulted in no clinical signs but measurable virus replication (nasal shedding and viraemia) which persisted for about 2 weeks post-infection. To evaluate the immunity conferred by EAV-G_{L}Δ immunization, immunized animals as well as EAV naive controls were infected with a virulent EAV challenge virus by the intranasopharyngeal route. Surprisingly, analysis of clinical signs, virus excretion and duration of viraemia between both groups of animals revealed that previous vaccination with a vaccine comprising a mutant EAV protected animals against disease upon infection with a virulent strain and reduced the level of its replication.

Also provided is a method for distinguishing unvaccinated animals or animals vaccinated with a vaccine according to the invention from animals infected with wild-type arterivirus or vaccinated with an unmodified arterivirus strain taking at least one serum sample from said animals and determining in said sample the presence of antibodies directed against an immunodominant epitope or marker expressed by said wild-type or unmodified arterivirus but not by said vaccine. For example, an animal belongs to the family of Equidae. In one embodiment of the invention, the presence of antibodies in a sample is determined in an ELISA or radioimmunoassay (RIA) assay. In a preferred embodiment, a distinction between different groups of animals is made by an immunoassay exploiting the modified (deleted or substituted) polypeptide or a part thereof as diagnostic antigen. A diagnostic antigen comprising a polypeptide or one or more parts thereof is prepared by standard methods, e.g. by heterologous expression or in vitro synthesis. For example, an ELISA test based on the immunodominant G_{L} protein is instrumental to distinguish animals vaccinated with a recombinant EAV in which at least part of the immunodominant G_{L} domain is deleted or replaced from animals infected or vaccinated with wild-type EAV. One of the formats evaluated in the present invention used as a suitable diagnostic antigen an ovalbumin-conjugated synthetic peptide comprising residues 81-106 of the G_{L} protein.

In a further embodiment, the invention provides an isolated or recombinant arterivirus nucleic acid, like a cDNA or an RNA, at least comprising a modification in a nucleic acid sequence encoding a viral polypeptide, said modification allowing to distinguish animals vaccinated with a vaccine from animals infected with wild-type arterivirus wherein said arterivirus is a constituent of said vaccine. A cDNA or an RNA obtained from a nucleic acid according to the invention is also provided. Also provided is a cell expressing a cDNA according to the invention. In a further embodiment, the invention provides a polypeptide encoded by a nucleic acid according to the invention. Such a modified arteriviral polypeptide or proteinaceous substance is for example advantageously used as constituent of a peptide vaccine. In an other embodiment, a polypeptide provided herein is used in a diagnostic test to determine the presence of an antibody directed against said polypeptide. The invention furthermore provides an isolated or recombinant arterivirus nucleic acid at least comprising a modification in a nucleic acid sequence encoding a viral polypeptide, wherein said nucleic acid is additionally provided with a nucleic acid encoding a heterologous antigen. For example, a nucleic acid according to the invention encoding an attenuated deletion virus can be used as therapeutic vector when it is additionally provided with genetic information specifying such therapeutic entity. In a preferred embodiment, said additional nucleic acid sequence encodes an immune-stimulatory protein or part thereof.

Further, the invention provides an antibody specifically reactive with an antigenic determinant present on a wild-type arterivirus polypeptide, said antigenic determinant not being present in vaccine according to the invention. Use of an antibody as provided, like a monoclonal antibody, for example allows to distinguish animals infected with wild-type arterivirus or animals vaccinated with an unmodified strain from animals vaccinated with a vaccine according to the invention. Also provided is a method for distinguishing animals vaccinated with a vaccine according to the invention from animals infected with wild-type arterivirus comprising taking at least one sample from said animal and determining in said sample the presence of antibodies specifically reactive with a polypeptide according to the invention.

In one embodiment, the invention provides a diagnostic kit for use in a method for distinguishing unvaccinated animals or animals vaccinated with a vaccine according to the invention from animals infected with wild-type arterivirus or vaccinated with an unmodified arterivirus strain, including an immunodominant epitope, or an antibody directed against said epitope, expressed by wild-type or unmodified arterivirus but not by a vaccine according to the invention. For example, a serological test kit is provided to discriminate between animals vaccinated with the EAV deletion mutant EAV-G_{L}Δ and animals infected or vaccinated with wild-type or unmodified EAV. Such a kit may comprise a diagnostic antigen e.g. an ovalbumin-conjugated synthetic peptide comprising residues 81-106 of the EAV G_{L} protein, a region deleted in EAV-G_{L}Δ, or a recombinantly produced protein 6hisG_{L}ecto(18-122), which comprises the whole putative ectodomain of the EAV G_{L} protein. As is shown in the detailed description, both antigens are useful to detect antibody responses to G_{L} ectodomain derived antigens by ELISA.

### Detailed description

### Example 1:

### Preparation and in vitro characterization of an EAV derivative lacking the predominant virus-neutralizing domain of the G_{L} protein.

### 1. Construction of a mutant EAV lacking residues 66 through 112 of the G_{L} protein (EAV-G_{L}Δ).

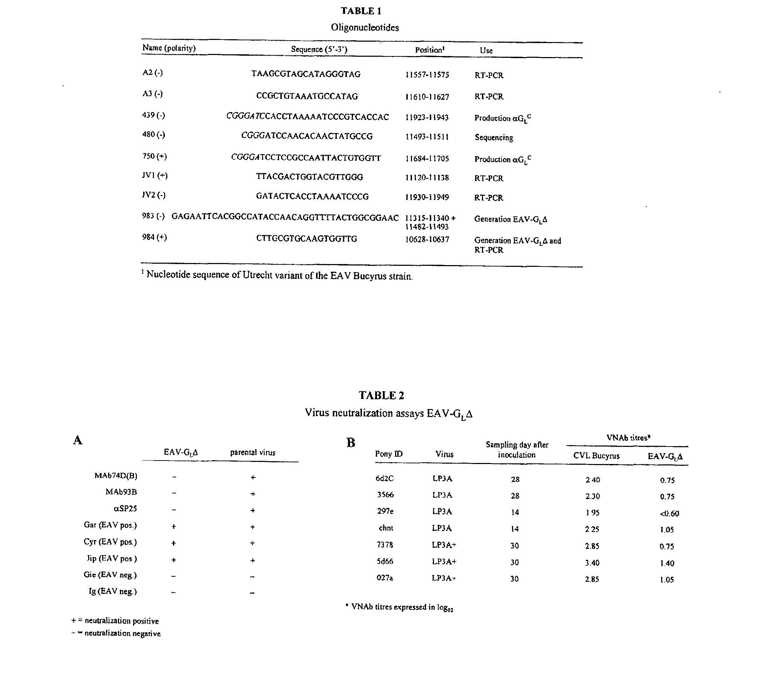

**Aim:** Preparation of a mutant EAV from which a 141 nucleotide segment encoding residues Cys⁶⁶ through Tyr¹¹² of the G_{L} protein have been deleted. **Procedure:** Plasmid pEAN515 is a derivative of EAV infectious cDNA clone pEAV030-*Bgl*II KO (de Vries et al., 2000), in which the cryptic transcription termination signal at position 8941-8956 was removed by site directed mutagenesis (mutations: A8941C, T8945A, C8946G, T8947C, T8950C, and G8956C) while simultaneously creating a *Msc*I site. The region of interest, i.e. the domain encoding the G_{L} protein, was modified using standard DNA techniques as follows. A PCR product was synthesized by PCR using the oligonucleotide 983, which represents EAV genomic nucleotides 11493-11481 + 11340-11315, and oligonucleotide 984, which corresponds to genomic nucleotides 10628-10637, and using pEAN515 as the DNA template. For primer specifications, see Table 1. The resulting 724 nucleotides long PCR fragment was treated with *Bgl*II and *Eco*RI, and the 638 bp digestion product was sequenced as well as cloned into pEAN515 after this plasmid had been digested with the same restriction enzymes. This resulted in the plasmid designated pEANG_{L}Δ. The plasmid was first checked by restriction enzyme digestion using *Bgl*II and *Eco*RI. The DNA fragment obtained had the predicted size, i.e. it was consistent with the deletion of the 141-nucleotide segment as judged from a comparison of its electrophoretic mobility with that of the restriction fragment similarly prepared with the parental plasmid pEAN515, as shown in Fig. 1.

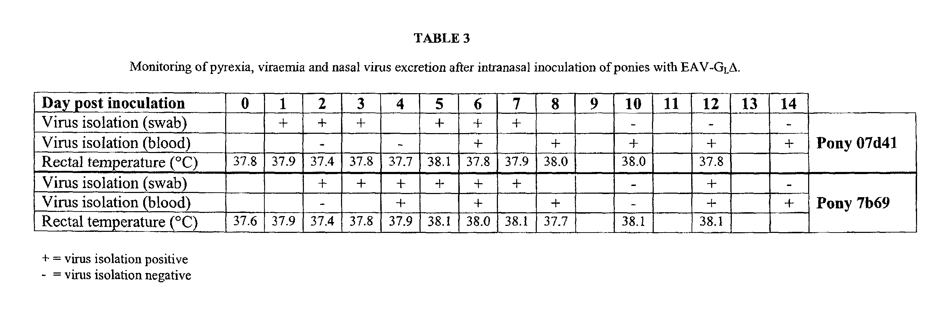

Subsequently, Baby Hamster Kidney cells (BHK-21 C13; American Type Culture Collection) were transfected with *in vitro* transcripts derived from pEANG_{L}Δ as well as from the parental cDNA construct, using procedures essentially as described by de Vries *et al.* (2000). After electroporation, clear cytopathic effect was observed in the cell cultures for both constructs indicating that the EAV-G_{L}Δ was viable. To investigate the presence and stability of the mutation, culture medium of the electroporated cells was inoculated onto a fresh culture of BHK cells and the next day the viral RNA was purified from the culture supernatant of the infected cells using QIAamp Viral RNA kit (QIAGEN). The genome segment containing the intended deletion was then sequenced. Thus, for reverse transcription the Superscript II Preamplification System (Gibco BRL Life Technologies) and primer A3, corresponding to genomic nucleotides 11627-11610, were used. Subsequently, the PCR reaction was carried out with primers 984 and A2 (nucleotides 11575-11557) and the PCR product was finally cloned into the pGEM®-T Easy vector (Promega) using a standard A-tailing protocol. The insert was analyzed by sequencing of the alkali-denatured plasmid DNA using a T7 DNA polymerase sequencing kit (Amersham Pharmacia Biotech), [α³³P]dATP (1.000 Ci/mmol; Amersham Pharmacia Biotech) and primer 480 (representing EAV genomic nucleotides 11511-11493). As is shown in Fig. 2, the sequence demonstrates that the deletion had been introduced into the viral genome. No other mutations could be observed in the PCR product.

**Conclusion:** EAV-G_{L}Δ, a deletion mutant lacking the G_{L} protein residues 66 through 112, is viable.

### 2. Characterization of the EAV-G_{L}Δ mutant.

### a. In vitro growth.

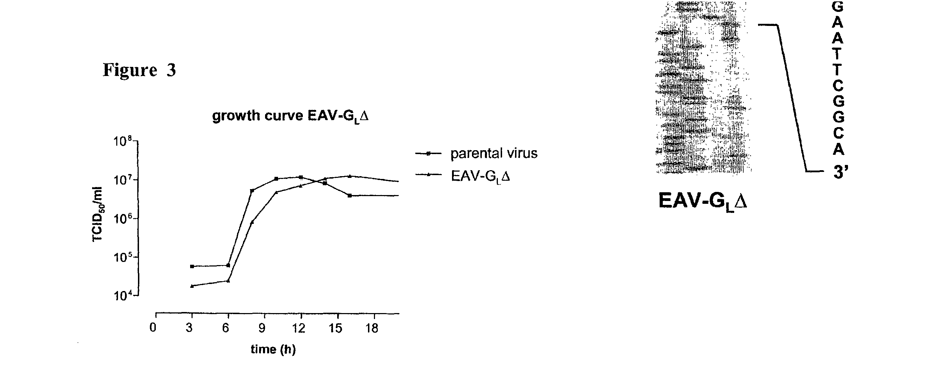

To investigate effects of the deletion in the G_{L} protein on mutant virus growth, BHK-21 cells were infected with the EAV-G_{L}Δ or the parental (i.e. the Utrecht variant of the Bucyrus strain) virus at high (>10) multiplicity of infection (m.o.i.). Subsequently, viral growth was monitored by taking samples from the culture supernatant at different time points and determining the viral titer. **Results:** As Fig. 3 illustrates, the EAV-G_{L}Δ mutant virus multiplied well, exhibiting a slightly decreased growth rate as compared to the parental virus. Whereas the parental virus reached its maximum titer at approximately 10 hours post-infection (p.i.), the deletion mutant reached its maximum titer approximately 4 hours later. Nevertheless, both viruses eventually reached similar maximal titers.

### b. Analysis of the G_{L} protein

Next we analyzed the mutation at the protein level by comparing the sizes of the G_{L} proteins of mutant and parental viruses. To this end radiolabeled viruses were prepared as follows. BHK-21 cells were mock-infected or infected at high m.o.i. with EAV-G_{L}Δ or the parental virus and labelled with [³⁵S]methionine using standard procedures (de Vries et al., 2000). Briefly, at 6 h p.i. the culture medium was replaced by prewarmed starvation medium. After a 30 min incubation at 39°C, 80 µCi of Redivue Pro-mix cell labeling mix [³⁵S]methionine (Amersham Pharmacia Biotech) was added and the cells were further incubated at 39°C. At 12 h p.i. the culture medium was harvested and cleared by centrifugation for 10 min at 4,000 rpm in an Eppendorf centrifuge. The supernatant was then mixed with one-fourth volume of 5 × lysis buffer (100 mM Tris-HCl [pH 7.6], 150 mM NaCl, 5% NP-40 (Nonidet P-40), 2.5% DOC (deoxycholate), 0.5% SDS (sodium dodecyl sulphate) containing 5 µg of aprotinin, leupeptin, and pepstatin A per ml) and supplemented with 100 mM N-ethylmaleimide (NEM). The lysate was cleared by centrifugation for 15 min at 14,000 rpm in an Eppendorf centrifuge. The pellet was discarded, and the supernatant supplemented with EDTA to a final concentration of 5 mM. Viral proteins were then subjected to immunoprecipitation with different antisera and analyzed using SDS-PAGE as described before (de Vries et al., 2000). The antibodies used were the following:
- anti-G_{L}^{C}: an antiserum directed against the carboxy terminus of the G_{L} protein; it was prepared by PCR amplification (using the primers 750 and 439 which correspond to genomic nucleotides 11684-11705 and 11943-11923, respectively) of the relevant region of EAV cDNA clone PB106 (de Vries et al., 1990) and cloning the *Bam*HI-digested PCR fragment into *Bam*HI-digested pQEX-3X. The nucleotide sequence of the PCR product was confirmed by sequencing. The EAV sequence was then expressed as a bacterial glutathione-S-transferase fusion protein designated FP55 in E.Coli and purified by preparative gel electrophoresis and elution from the gel using standard procedures. Finally, a New Zealand White rabbit was immunized with the fusion protein and sera were prepared as described (Wieringa et al., 2002).
- anti-SP25: a rabbit antipeptide serum directed to amino acids 75 through 95 of the EAV G_{L} protein (de Vries et al., 1995)
- MAb74D(B): an EAV G_{L}-specific murine monoclonal antibody (MAb) generated by Glaser et al. (1995).
- anti-M: a rabbit antipeptide serum (SP06) directed to the carboxy terminus of the EAV M protein described by de Vries et al.(1992)

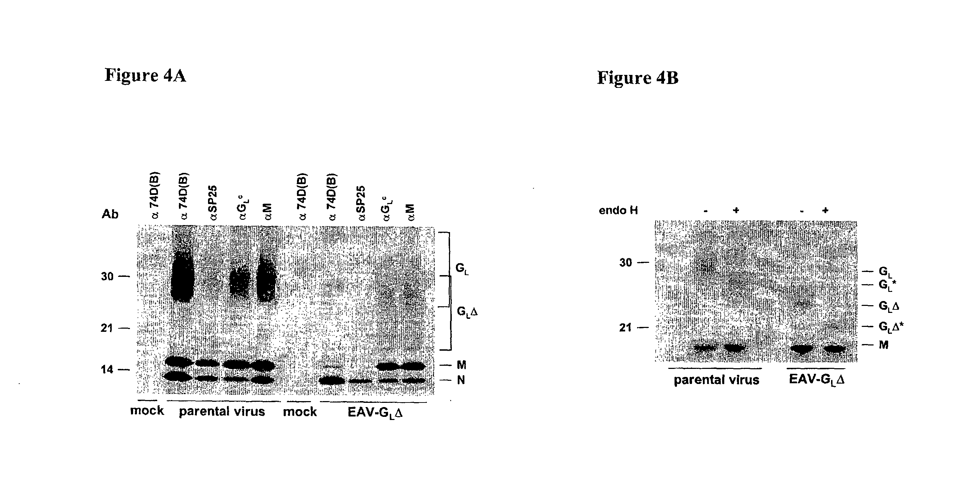

**Results:** An analysis of the G_{L} proteins of the two viruses is shown in Fig. 4A. As expected the G_{L} protein of the wild-type (WT) virus was precipitated by each G_{L} specific antibody. The protein appeared in gel as a characteristic smear of about 30-38 kDa. The G_{L}Δ protein was also seen as a smear but with a slightly increased mobility. After immunoprecipitation with the anti-G_{L}^{C} serum it migrated with an apparent mobility of about 28-34 kDa. In addition, a protein of 19 kDa was immunoprecipitated which, as discussed below, probably represents unglycosylated G_{L}Δ protein. As expected, the G_{L}Δ protein was not immunoprecipitated using MAb 74D(B) and serum anti-SP25, since these antibodies recognize the deleted region. Further, the coprecipitation of the G_{L}Δ and M protein using M- and G_{L}-specific antisera, respectively, demonstrated that the G_{L}Δ and M proteins form covalently linked heterodimers as the wild-type proteins do (de Vries et al., 1995). The origin of the 17-kDa protein that comigrates with the M protein in the immunoprecipitate of the EAV-G_{L}Δ prepared using MAb74D(B) is unknown.

It is unlikely to represent the M protein because neither the SP25 antiserum nor the MAb 74D(B) recognizes the G_{L}Δ protein, excluding the possible coprecipitation of the M protein with the G_{L}Δ protein. Most likely the protein is nonspecifically precipitated by MAb74D(B), since a protein of similar size is often also precipitated using different preimmune sera (Wieringa et al, 2002). The approximately 13 kDa protein seen in all immunoprecipitates from both infected cultures is the N protein that is known to be non-specifically precipitated.

Because of the diffuse appearance of the two G_{L} proteins, which is due to their heterogeneous glycosylation, a clear size discrimination is difficult. To allow a better comparison of their polypeptide moieties we repeated a similar [³⁵S]methionine radiolabeling of EAV-G_{L}Δ and wild-type EAV infected BHK-21 cells but now in the presence of 1 mM 1-Deoxymannojirimycin (DMJ; Boehringer Mannheim) to prevent maturation of the N-linked oligosaccharides. Viral particles in the culture supernatants were solubilized and subjected to immunoprecipitation as above using the anti-G_{L}^{C} serum or the corresponding preserum. The immunoprecipitates obtained were treated with Endoglycosidase H (endo H) or mock-treated: washed immunoprecipitates were resuspended in 20 µl denaturation buffer (0.5% SDS, 1% β-mercaptoethanol) containing 2 mM PMSF (phenylmethylsulfonyl fluoride) and 1/10 volume of 10 × G5 buffer (New England Biolabs_{Inc}) and 500 U of Endoglycosidase H (New England Biolabs_{Inc}) were added. The enzyme was omitted in the control. After an incubation at 37°C for 3 h, the samples were further processed for electrophoresis as described above.

**Results:** When synthesized in the presence of DMJ, it is clear that the G_{L}Δ protein is significantly smaller than the parental protein (Fig. 4B). The mutant protein migrated with an apparent molecular mass of about 24 kDa, clearly ahead of the approximately 30kDa wild-type glycoprotein. The Endo H treatment reduced the apparent sizes of both proteins with 2-3 kDa to approximately 27 and 22 kDa, respectively. Obviously, the deletion of residues 66-112 reduced the apparent molecular mass of the unglycosylated G_{L} protein with approximately 5 kDa. The results also confirm that in virions the G_{L}Δ protein is glycosylated and indicate that the apparent molecular mass of the unglycosylated protein is close to prediction.

**Conclusions:** The targeted deletion of the immunodominant region of the G_{L} protein was successfully achieved, as confirmed by genetical, immunological, biochemical and electrophoretic analyses. The mutant virus seems not to be significantly impaired in its in vitro growth properties.

### c. Lack of neutralization of EAV-G_{L}Δ by antibodies to the deleted domain

To study the effect of the deletion on virus neutralization *in vitro,* a virus neutralization assay was performed using MAb's 93(B) and 74D(B), the rabbit antipeptide serum αSP25, three horse sera positive for anti-EAV antibodies as well as two seronegative horse sera. MAb 93(B) and 74D(B) have been shown to recognize epitopes in the deleted amino acid 66-112 sequence of the G_{L} protein (Glaser et al., 1995).

**Procedure:** 100 TCID₅₀ units of wild-type EAV or mutant EAV-G_{L}Δ were mixed with tissue culture supernatant of hybridoma's producing MAb 93(B) or 74D(B), with serum from three horses seropositive for EAV or with serum from two EAV-seronegative horses in 96-well microtitre plates. The plates were then incubated for 1h at room temperature, after which guinea pig complement (SIGMA) was added to a final concentration 10%. After another 1-h incubation at 37°C approximately 1 × 10⁵ BHK-21 C13 cells were added. Individual wells were scored for cytophatic effect following a 48-120 h incubation at 37°C. Each individual virus neutralization assay was performed in triplicate and contained a virus control to ensure that 100 TCID₅₀ of the Bucyrus strain of EAV was added to each well, and series of BHK-21 cell controls.

**Results:** The combined results of the virus neutralization assays are compiled in Table 2A. It turned out that the EAV seropositive horse sera were able to neutralize the EAV-G_{L}Δ as well as the parental virus. In contrast, however, the MAb's were unable to neutralize the EAV-G_{L}Δ virus while the parental virus was neutralized efficiently. Neither of the EAV seronegative sera was capable of neutralizing any of the viruses.

**Conclusion:** Clearly, the deletion renders the mutant virus insensitive to the antibodies directed against the deleted region. These viruses remain, however, sensitive to neutralization by the polyclonal anti-EAV antibodies.

**Procedure:** In another experiment we comparatively analyzed the virus neutralizing antibody titers of a panel of sera collected from horses experimentally infected with the virulent EAV strain LP3A and its single passage derivative LP3A+ (for details about this virus: see below). These sera were titrated against EAV-G_{L}Δ and EAV strain CVL Bucyrus, which has a wild-type G_{L} protein, as described before (Castillo-Olivares et al., 2001). **Results:** As Table 2B shows, all sera neutralized the CVL Bucyrus virus much more efficiently than the deletion virus EAV-G_{L}Δ, which was neutralized relatively poorly.

Conclusion: The results are consistent with the deletion virus having lost the major part of its neutralizing epitopes.

### Example 2

### Vaccination of horses with EAV-G_{L}Δ virus and challenge infection with a virulent EAV

### a. Preparation of mutant virus inoculum

The EAV-G_{L}Δ virus had been generated in non-equine cells. In order to prepare a stock for immunization, this virus was passaged once in primary equine embryonic lung cells (EELs, fibroblastoid). Briefly, EELs were infected at an m.o.i. of approx. 0.002 and incubated for 72 hours at 37°C, 5% CO₂, using MEM (Eagle's minimum essential medium [Sigma, M2279], sodium bicarbonate buffered, supplemented with antibiotics) with 10% foetal bovine serum. Following the incubation, the tissue culture fluid was harvested, freeze/thawed, cell debris was removed by centrifugation and the supernatant stored as aliquots at -70°C.

Prior to use, this virus stock was analyzed by RT-PCR, using ORF 5 specific primers, to confirm that it consisted of virus with an internal deletion in ORF5. Thus, viral RNA was purified from the cleared tissue culture fluid using the High Pure PCR Template Prep. Kit (Roche Molecular Biologicals) following the manufacturer's instructions. Briefly, 200µl of tissue culture fluid were mixed with 200 µl of 'Binding Buffer' (6M guanidine-HCl, 10mM urea, 10mM Tris-HCl, 20% Triton-X-100, pH4.4) and 40 µl of Proteinase K and incubated for 10 min at 72°C. After the addition of 100 µl of isopropanol, the viral RNA was bound to a glass fiber filter and washed twice with 500 µl of washing buffer (20mM NaCl, 2 mM Tris-HCl, 80% ethanol, pH 7.5). Finally the RNA was eluted with 200µl of 10mM Tris-HCl, pH 8.5. As a control, RNA was similarly purified from culture fluid of RK13 cells infected with the CVL Bucyrus strain of EAV. Reverse transcription of viral RNA and subsequent amplification of cDNA of EAV ORF5 were performed in a one step reaction using the *rTth* DNA Polymerase and EZ Buffer (Perkin Elmer) in the presence of manganese acetate Mn(OAc)₂ with primers JV1 and JV2. Five µl of purified viral RNA solution was included in a 50µl PCR reaction containing 300 µM of each dNTP's, 0.1 units/µl of the enzyme, 0.4µM of each primer and 2.5 mM Mn(OAc)₂ diluted in EZ buffer.

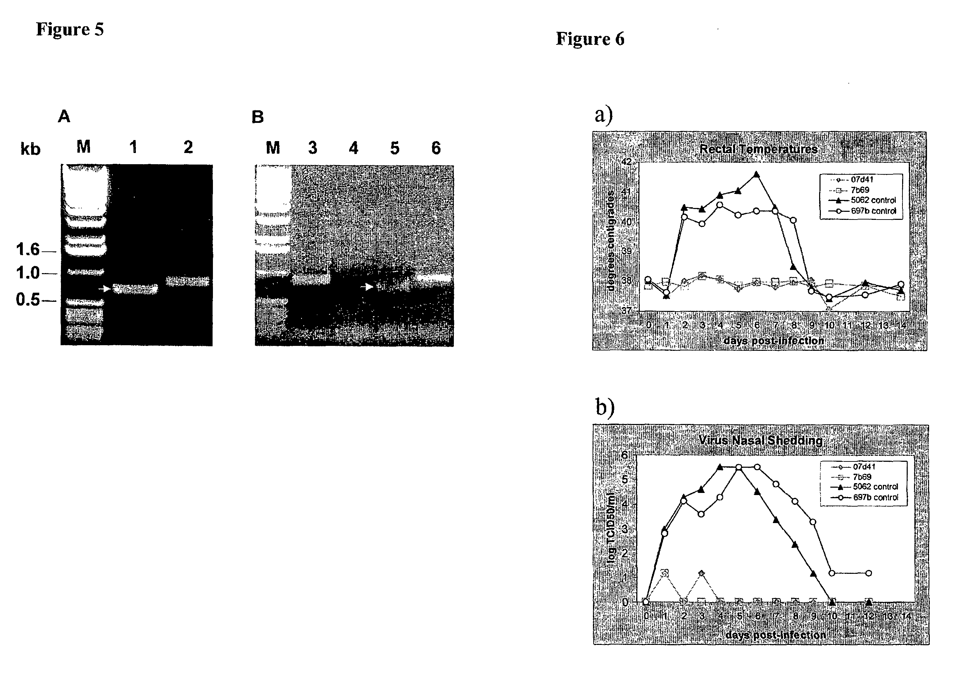

**Results:** As shown in Fig. 5A, the EAV-G_{L}Δ RT-PCR product (lane 1) is approximately 150bp smaller than the wild type ORF5 product from the CVL Bucyrus strain (lane 2). The size difference is consistent with the 141-nucleotide deletion.

### b. Vaccination with EAV-G_{L}Δ and evaluation of clinical, virological and serological responses

**Procedures** for EAV infection of ponies have been described previously (Castillo-Olivares *et al,* 2001). Four year-old castrated male Welsh Mountain Ponies (5062, 697b, 7b69 and 07d41), seronegative to EAV, were used in this study. Ponies 7b69 and 07d41 were stabled in a contained environment facility and inoculated with 10⁶ TCID₅₀ of EAV-G_{L}Δ by the intranasopharyngeal route and monitored closely for 28 days. Nasopharyngeal swabs, heparinised blood and serum samples were collected at regular intervals and analysed for virus and antibody detection.

Virus isolation from blood and from nasopharyngeal samples, and determination of virus neutralizing (VN) antibody responses were conducted as previously described (Castillo-Olivares *et al.,* 2001). The VN test was performed using three different EAV strains: LP3A+, Bucyrus CVL and EAV-G_{L}Δ. The Bucyrus CVL EAV strain is the standard reagent for EAV serological testing. The ORF5 sequence of Bucyrus CVL differs at a number of positions from that of LP3A+, but shows a high degree of antigenic cross-reactivity. Antibody responses to two G_{L} derived antigens were analysed by ELISA following the procedures described by Nugent *et al.* (1999) with minor modifications. The antigens chosen were an ovalbumin-conjugated synthetic peptide (G_{L}-OVA) comprising amino acids 81-106, a region deleted in EAV-G_{L}Δ, and the recombinant protein G_{L}-6His (18-122) which comprises the whole putative ectodomain of the EAV G_{I,} protein.

**Results:** No clinical signs were observed in ponies 7b69 and 07d41 following EAV-G_{L}Δ infection. Accordingly, rectal temperatures remained normal (Table 3). However, cell associated viraemia and nasopharyngeal virus excretion were observed in both ponies (Table 3). Viraemia, referring to the presence of infectious virus in the blood, was first detected on days 4 and 6 for ponies 7b69 and 07d41, respectively, and lasted until day 14 in both animals (viraemia was not detected on days 16, 19 and 21, data not shown). Virus excretion from the upper respiratory tract occurred between days 1 and 7 but the infectivity was below 10^{1.5} TCID₅₀/ml except for the sample collected on day 5 from pony 07d41 (10^{1.5} TCID₅₀/ml). RT/PCR analysis of viral RNA extracted from the virus isolated from a blood sample collected from pony 7b69 on day 4 post-inoculation confirmed that the recovered virus carried the expected internal deletion of ORF5. This is shown in Fig. 5B where RT-PCR products prepared using primers JV1 and JV2 with the following viruses are compared: wild type EAV strain CVL Bucyrus grown in RK13 cells (lane 3); virus recovered in RK13 cells from EAV-G_{L}Δ infected pony (lane 5); challenge virus (LP3A+) prepared in EEL cells (lane 6); uninfected RK13 tissue culture fluid (lane 4). In the lanes marked M a mix of marker DNAs was analysed for reference (sizes of relevant marker DNAs are indicated at the left).

Both ponies developed good levels of neutralising antibody against the immunising virus in their serum, but the neutralizing activity against LP3A+ or CVL Bucyrus was weak (described in detail below).

**In conclusion:** Immunisation with EAV-G_{L}Δ resulted in no clinical signs, but measurable virus replication (nasal shedding and viraemia) which persisted for 2 weeks post-infection.

### c. Challenge infection with EAV LP3A+ and evaluation of clinical, virological and serological responses

**Procedure:** The above ponies were released from containment to an isolated barn when they were no longer infectious. Forty six days after the immunisation ponies 7b69 and 07d41 and EAV seronegative ponies 5062 and 697b were inoculated with 10^{6.1} TCID₅₀ units of the virulent EAV strain LP3A+ by the intranasopharyngeal route. All four ponies were clinically inspected twice a day and serum, heparinised blood and nasopharyngeal swabs were collected at regular intervals until the end of the study. The ponies were released to a third isolated barn when they were regarded as non-infectious, prior to then being transferred 2 weeks later to an isolated paddock for 4 weeks before they were allowed to have contact with any other equines.

**Results:** Both control ponies developed evident clinical signs of EAV infection. Their symptoms included pyrexia, depression, anorexia, conjunctivitis, nasal discharge and ataxia, the well-known sequelae of an evident EAV infection. In contrast, the vaccinated ponies 07d41 and 7b69 remained asymptomatic during the entire study period. The rectal temperatures measured during the first 2 weeks were fully consistent with these observations. As diagrammed in Fig. 6 high fever developed in the control ponies during the second day p.i. and remained for almost a week, while no obvious elevation in body temperature was observed in the vaccinated animals. Viraemia was obvious in the control ponies from day 2 lasting up to day 21 p.i. whereas virus was detected only on day 8 in blood from both vaccinated ponies (data not shown). Virus was excreted from the upper respiratory tract of the control animals during the first 10-12 days, peaking around day 5 p.i. (Fig. 6); viral infectivities in the nasal swab extracts were very high (>10³ TCID₅₀/ml) between days 2-9 (#697b) and 2-7 (#5062), reaching maximum levels exceeding 10⁵ TCID₅₀/ml. In contrast, for the immunized ponies virus was only isolated on one or two occasions from ponies 7b69 and 07d41, respectively, and the infectivity remained below 10^{1.5} TCID₅₀.

**Conclusion:** Previous vaccination with the deletion mutant EAV-G_{L}Δ clearly protected the animals against disease upon infection with a virulent EAV strain and reduced the level of its replication. Surprisingly, the lack of the immunodominant G_{L} domain does not interfere with the vaccine virus' ability to provide protection, presumably because it induces effective immune responses other than virus neutralizing antibody, such as cell-mediated immunity (e.g. cytotoxic T lymphocytes).

### d. Neutralizing antibody responses to vaccination and challenge infection.

Using the sera collected throughout the vaccination/challenge experiment the development of virus neutralizing activity in each individual animal was determined. To evaluate the effect of the lack of the immunodominant region of G_{L} in the vaccine virus we analyzed the virus-neutralizing antibody (VNAb) titres not only against EAV-G_{L}Δ but also against EAV strains LP3A+ and Bucyrus CVL that carry wild-type G_{L} proteins. The assay was carried out as described before (Castillo-Olivares et al., 2001).

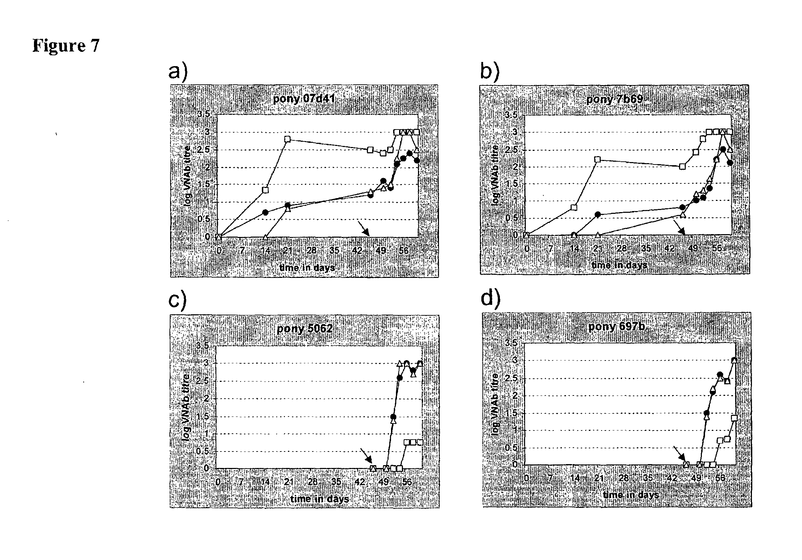

**Results:** Following immunization, ponies 7b69 and 07d41 developed a weak VNAb response against the Bucyrus CVL and LP3A+ strains (Fig. 7). By day 14 post-infection, the neutralizing antibodies were detectable only against the LP3A+ strain for a single pony. The VNAb titres increased slightly until the day of the challenge (day 46) when the titres for both EAV wild type strains were detectable in both ponies immunized with EAV-G_{L}Δ. In contrast, the neutralizing antibody response was much stronger against EAV-G_{L}Δ, reaching titres of 1.35 and 0.8 by day 14 post-infection and 2.2 and 2.8 on day 21 (for ponies 7b69 and 07d41, respectively). At the time of challenge, VNAb titres for both immunized ponies were at least ten times higher against EAV-G_{L}Δ than against the wild-type strains.

After challenge, both immunized ponies responded with a rapid increase in neutralizing antibodies to all three viruses, with titres being generally higher against EAV-G_{L}Δ. The control ponies showed a typical VNAb response after EAV infection against both wild type viruses, with titres detectable by day 6 and increasing rapidly to reach high values by day 14. In contrast, the titres against the EAV-G_{L}Δ virus were very low.

**Conclusion:** Infection of ponies with EAV-G_{L}Δ elicits antibody responses that neutralize wild-type EAV strains poorly. Yet, this infection protects effectively against disease after subsequent inoculation with such viruses.

### e. Evaluation of G_{L}-specific antibody responses: serological discrimination between vaccinated and infected animals.

**Aim:** The application of EAV-G_{L}Δ as a marker vaccine requires that infected animals can be identified irrespective of their vaccination status.

**Procedure:** The availability of G_{L}-based ELISA's that we established recently (Nugent et al., 2000) allowed us to analyse the antibody responses directed against the entire G_{L} ectodomain (residues 18-122) and against part (residues 81-106) of the domain deleted in EAV-G_{L}Δ (residues 66-112). The former test uses as an antigen a His-tagged bacterial expression product (G_{L}-6His) while in the latter assay an ovalbumin-coupled synthetic peptide (G_{L}-OVA) is applied to the ELISA plates. Selected serum samples from the four ponies were analysed in parallel with a positive control serum taken from a pony that had been immunized twice with the G_{L}-6His protein (pony p6, 2 weeks post-vaccination with G_{L}-6His antigen; Castillo-Olivares et al., 2001).

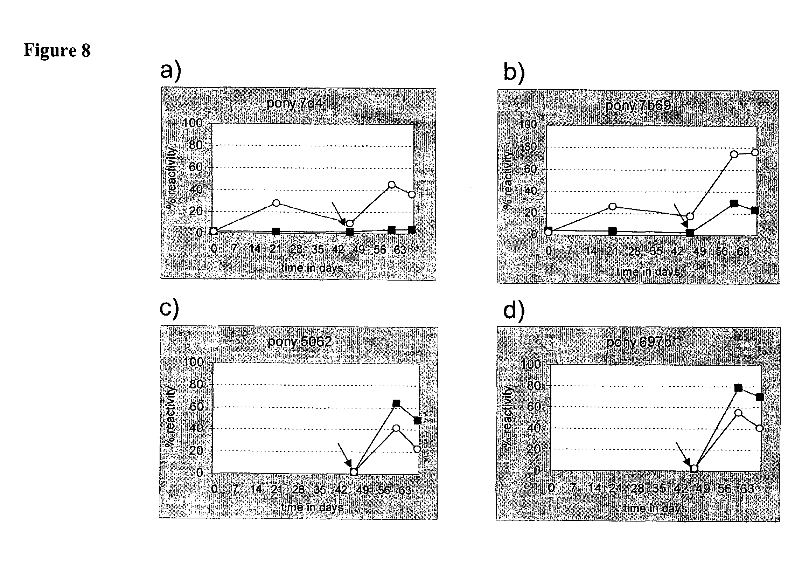

**Results:** The observations, expressed as the percentages of the ELISA reactivities with respect to the control, are compiled in Fig. 8. As expected, the vaccination of ponies 7b69 and 07d4 with EAV-G_{L}Δ had not induced a detectable antibody response against the deleted G_{L} domain. Reactivity against G_{L}-OVA became measurable only after the challenge infection with wild-type EAV, particularly in the non-vaccinated animals. In contrast, both vaccinated ponies developed an antibody response to G_{L}-6His, which was subsequently boosted after challenge becoming at least three-fold higher than that against G_{L}-OVA. In the two control ponies, however, the challenge infection induced antibody responses against the two antigens that, in contrast to the pre-immunised ponies, were at similar levels for both antigens. **Conclusion:** A serological assay based on the immunodominant domain deleted from EAV-G_{L}Δ allows discrimination of animals infected with this virus from those infected with wild-type viruses.

### Figure legends

FIG. 1. **Restriction enzyme analysis of the pEANG**_{**L**}**Δ construct**. Plasmids pEAN515 and pEANG_{L}Δ were treated with *Bgl*II and *Eco*RI and the products were analysed by agarose gel electrophoresis. Numbers on the right refer to the anticipated size of the specific digestion products. On the left the positions and sizes of marker DNA fragments are indicated (in nucleotides).
FIG. 2. **RT-PCR analysis of the EAV-G**_{**L**}**Δ virus**. Viral RNA was purified from culture supernatants of infected cells. cDNA synthesis was primed with primer A3 (-); for PCR amplification primers 984 (+) and A2 (-) were used. The RT-PCR product was cloned into the pGEM® -T Easy vector and sequenced. The deduced nucleotide sequence of the relevant part of EAV-G_{L}Δ ORF5 is shown.
FIG. 3. **One-step growth curves of the parental and the EAV-G**_{**L**}**Δ viruses in BHK-21 cells**. BHK-21 C13 cells were infected in parallel at high m.o.i with the EAV-G_{L}Δ mutant and the parental virus. Virus titers in the culture medium were monitored by taking aliquots at different time points during infection and TCID₅₀ titration of the viruses. EAV-G_{L}Δ is indicated by black triangles; the parental virus is indicated by black squares.
FIG. 4. **Immunoprecipitation analysis of EAV-G**_{**L**}**Δ**. EAV-G_{L}Δ infected, parental virus infected and mock infected BHK-21 C13 cells were labeled with [³⁵S]methionine from 6¹/₂ h to 12 h post-infection either in the absence (A) or presence of DMJ (B). After removal of cell debris by low-speed centrifugation, the labeled virus present in the cell culture medium was dissolved in lysis buffer containing 20 mM NEM and subjected to immunoprecipitation with the MAb 74D(B) , with the G_{L}-peptide antiserum anti-SP25, with the anti-G_{L}^{C}-serum against the G_{L} protein's carboxy terminus, or with the M protein-specific antipeptide serum anti-M (directed against peptide SP06). In panel B the samples were immunoprecipitated with anti-G_{L}^{C}. The immunoprecipitates in (B) were treated (+) or mock-treated (-) with endo H (endo H). Samples were finally dissolved in LSB containing 50 mM DTT and analysed by SDS-15% PAGE. Numbers on the left refer to positions of the molecular weight markers (in kilodaltons). At the right are indicated the positions of the viral proteins; for panel (B): G_{L} and G_{L}Δ indicate the glycosylated forms of the G_{L} and G_{L}Δ proteins while G_{L}* and G_{L}Δ* refer to their deglycosylated forms.
FIG. 5: RT-PCR analysis of ORF5 from EAV-G_{L}Δ inoculated into and retrieved from ponies. RT-PCR amplification of ORF5 was carried out on tissue culture fluid, using primers JV1 and JV2. The presence of the deletion in ORF5 was checked both of the input virus and of virus present in the blood of one of the ponies (#7b69) at day 4 p.i. with EAV-G_{L}Δ. Lane 1: EAV-G_{L}Δ stock virus prepared in EEL cells; lanes 2 and 3: wild type EAV strain CVL Bucyrus grown in RK13 cells; lane 4: uninfected RK13 tissue culture fluid; lane 5: virus recovered in RK13 cells from EAV-G_{L}Δ infected pony; lane 6: challenge virus (LP3A+) prepared in EEL cells. In the lanes marked M a mix of marker DNAs was analysed for reference (sizes of relevant marker DNAs are indicated at the left).
FIG. 6: Pyrexia (a) and nasal virus excretion (b) determined over a 14 day period for pre-vaccinated (#7b69 and 07d41) and control ponies (#5062 and 697b) following intranasopharyngeal challenge with the LP3A+ virus strain.
FIG. 7: Virus neutralising antibody responses in serum of ponies 7b69, 07d41, 5062 and 697b against EAV-G_{L}Δ (open squares), LP3A+ (filled circles) and Bucyrus CVL (open triangles) after intranasopharyngeal vaccination (ponies 7b69 and 07d41) with EAV-G_{L}Δ (day 0) and intranasopharyngeal challenge infection with EAV LP3A+ (day 46; indicated by arrow).
FIG. 8: G_{L} protein specific antibody responses of ponies 7b69, 07d41, 5062 and 697b after intranasopharyngeal immunization (ponies 7b69 and 07d41) with EAV-G_{L}Δ (day 0) and intranasopharyngeal challenge infection with EAV LP3A+ (day 46; indicated by arrow) measured by ELISA against antigens G_{L}-6His (G_{L} residues 18-122) and G_{L}-OVA (G_{L} residues 81-106). Reactivities of sera are expressed with respect to a positive control serum sample obtained from pony p6, 2 weeks post-vaccination with G_{L}-6His antigen (Castillo-Olivares et al., 2001). Reactivities against the G_{L}-6His antigen are represented by the open circles, those against G_{L}-OVA by the filled squares.

### Table 1: Oligonucleotide primers

### Table 2

Compilation of results of virus neutralization assays.

### Table 3

Pyrexia, virus nasal excretion and viraemia from ponies 7b69 and 07d41 measured over a 14 day period following intranasal infection with EAV-G_{L}Δ.

### References

Balasuriya, U. B. R., J. F. Patton, P. V. Rossitto, P. J. Timoney, W. H. McCollum, and N. J. MacLachlan. 1997. Neutralization determinants of laboratory strains and field isolates of equine arteritis virus: identification of four neutralization sites in the amino-terminal ectodomain of the G_{L} envelope glycoprotein. Virology 232:114-128

Castillo-Olivares J, de Vries AA, Raamsman MJ, Rottier PJ, Lakhani K, Westcott D, Tearle JP, Wood JL, Mumford JA, Hannant D, Davis-Poynter NJ. 2001. Evaluation of a prototype sub-unit vaccine against equine arteritis virus comprising the entire ectodomain of the virus large envelope glycoprotein (G(L)): induction of virus-neutralizing antibody and assessment of protection in ponies. J Gen Virol 82:2425-2435

Dea S. C.A. Gagnon, H.Mardassi, B.Pirzadeh, and D.Rogan. 2000. Current knowledge on the structural proteins of porcine reproductive and respiratory syndrome (PRRSV) virus: comparison of the North American and European isolates. Arch.Virol. 145:659-688.

De Vries, A.A.F., E.D.Chirnside, P.J.Bredenbeek, L.A.Gravestein, M.C.Horzinek, and W.M.J.Spaan. 1990. All subgenomic mRNAs of equine arteritis virus contain a common leader sequence. Nucl.Acids Res. 18:3241-3247.
de Vries, A. A. F., E. D. Chirnside, M. C. Horzinek, and P. J. M. Rottier. 1992. Structural proteins of equine arteritis virus. J. Virol. 66:6294-6303
de Vries, A. A. F., S. M. Post, M. J. B. Raamsman, M. C. Horzinek, and P. J. M. Rottier. 1995. The two major envelope proteins of equine arteritis virus associate into disulfide-linked heterodimers. J. Virol. 69:4668-4674.
de Vries, A. A. F., M. C. Horzinek, P. J. M. Rottier, and R. J. de Groot. 1997. The genome organization of the Nidovirales: similarities and differences between arteri-, toro-, and coronaviruses. Semin. Virol. 8:33-47.
de Vries, A.A.F., A. L. Glaser , M. J. B. Raamsman , C. A. de Haan , S. Sarnataro , G. J. Godeke and , P. J. M. Rottier. 2000. Genetic manipulation of equine arteritis virus using full-length cDNA clones: Separation of overlapping genes and expression of a foreign epitope. Virology 270:84-97.

Glaser, A. L., A. A. F. de Vries, and E. J. Dubovi. 1995. Comparison of equine arteritis virus isolates using neutralizing monoclonal antibodies and identification of sequence changes in G_{L} associated with neutralization resistance. J. Gen. Virol. 76:2223-2233

Glaser, A. L., E. D. Chirnside, M. C. Horzinek, and A. A. F. de Vries. 1997. Equine arteritis virus. Theriogenology 47:1275-1295.

Nugent, J., Sinclair, R., de Vries, A. A. F., Eberhardt, R. Y., Castillo-Olivares, J., Davis-Poynter, N., Rottier, P. J. M. & Mumford, J. A. 2000. Development and evaluation of ELISA procedures to detect antibodies against the major envelope protein G_{L} of equine arteritis virus. J. Virol. Methods 90:167-183

Plageman P.G.W. and V.Moenig. 1992. Lactate dehydrogenase-elevating virus, equine arteritis virus, and simian hemorrhagic fever virus: A new group of positive-strand RNA viruses. Adv. Virus Res. 41:99-192.

Snijder, E. J., and J. J. M. Meulenberg. 1998. The molecular biology of arteriviruses. J. Gen. Virol. 79:961-979

Wieringa R., A.A.F. de Vries, M.J.B. Raamsman, and P.J.M. Rottier. 2002. Characterization of two new structural glycoproteins, GP₃ and GP₄, of equine arteritis virus. J.Virol. 76:10829-10840.

## Claims

1. A vaccine directed against infection or disease caused by an arterivirus, said vaccine comprising an arterivirus provided with a modification in a nucleic acid sequence allowing to distinguish animals vaccinated with said vaccine from animals infected with wild-type arterivirus.

2. A vaccine according to claim 1 wherein said modification comprises deletion of at least one nucleic acid.

3. A vaccine according to claim 1 wherein said genetic modification comprises substitution of at least one nucleic acid.

4. A vaccine according to any one of claims 1 to 3 wherein said nucleic acid sequence encodes a viral envelope protein.

5. A vaccine according to claim 4 wherein said nucleic acid sequence encodes the ectodomain of said envelope protein.

6. A vaccine according to claim 1-5 wherein said arterivirus is Equine Arteritis Virus (EAV).

7. A vaccine according to claim 6 wherein said nucleic acid sequence encodes the G_{L} protein.

8. A vaccine according to claim 7 wherein said modification comprises deletion of the nucleic acid sequence encoding the amino acid residues 66 to 112 of said G_{L} protein.

9. A vaccine according to claim 1-5 wherein said arterivirus is Porcine Reproductive and Respiratory Syndrome Virus (PRRSV).

10. A vaccine according to claim 1-5 wherein said arterivirus is Simian Hemorrhagic Fever Virus (SHFV).

11. A vaccine according to claim 9 or 10 wherein said nucleic acid sequence encodes the GP5 protein.

12. A vaccine according to claim 1-5 wherein said arterivirus is Lactate Dehydrogenase-elevating Virus (LDV).

13. A vaccine according to claim 12 wherein said nucleic acid sequence encodes the VP-3P protein of LDV.

14. A live attenuated vaccine according to any one of claims 1 to 13.

15. A killed or inactivated vaccine according to any one of claims 1 to 13.

16. A composition at least comprising an effective amount of vaccine according to any one of claims 1 to 15 which lowers the average clinical symptoms of a group of animals inoculated with said composition, then subsequently challenged with virulent arterivirus, relative to a group of identically challenged animals not inoculated with the composition.

17. A method for distinguishing unvaccinated animals or animals vaccinated with a vaccine according to any one of claims 1 to 15 from animals infected with wild-type arterivirus or vaccinated with an unmodified arterivirus strain comprising taking at least one sample from said animal and determining in said sample the presence of antibodies directed against an immunodominant epitope or marker expressed by said wild-type or unmodified arterivirus but not by said vaccine.

18. A method according to claim 17 wherein said animal belongs to the family of Equidae.

19. A method according to claim 17 or 18 wherein the presence of antibodies in a sample is determined in an ELISA or radioimmunoassay (RIA) assay.

20. A diagnostic kit for use in a method according to any one of claims 17 to 19, including an immunodominant epitope, or an antibody directed against said epitope, expressed by wild-type or unmodified arterivirus but not by a vaccine according to any one of claims 1 to 15.

21. An isolated or recombinant arterivirus nucleic acid at least comprising a modification in a nucleic acid sequence encoding a viral polypeptide, said modification allowing to distinguish animals vaccinated with a vaccine from animals infected with wild-type arterivirus wherein said arterivirus nucleic acid is a constituent of said vaccine.

22. An RNA or cDNA obtained from a nucleic acid according to claim 21.

23. A polypeptide encoded by a nucleic acid according to claim 21.

24. An antibody specifically reactive with an antigenic determinant present on a wild-type arterivirus polypeptide, said antigenic determinant not being present in vaccine according to any one of claims 1 to 15.

25. Use of an antibody according to claim 24 for distinguishing animals vaccinated with a vaccine according to claims 1 to 15 from animals infected with wild-type arterivirus.

26. A method for distinguishing animals vaccinated with a vaccine according to claims 1 to 15 from animals infected with wild-type arterivirus comprising taking at least one sample from said animal and determining in said sample the presence of antibodies specifically reactive with a polypeptide according to claim 23.

27. A nucleic acid according to claim 21 or 22 additionally provided with a nucleic acid encoding a heterologous antigen.

28. A nucleic acid according to claim 27 additionally provided with a nucleic acid encoding an immune-stimulatory protein or part thereof.


SEQUENCE LISTING

<110> Universiteit Utrecht

<120> Arterivirus marker vaccine

<130> P61886EP00

<140> EP 03075117.6
<141> 2003-01-14

<160> 9

<170> PatentIn version 3.1

<210> 1
<211> 19
<212> DNA
<213> Artificial Sequence

<220>
<223> oligonucleotide primer
<400> 1
taagcgtagc atagggtag 19

<210> 2
<211> 18
<212> DNA
<213> Artificial Sequence

<220>
<223> oligonucleotide primer
<400> 2
ccgctgtaaa tgccatag 18

<210> 3

<211>  29

<212>  DNA

<213>  Artificial Sequence

<220>

<223>  oligonucleotide primer

<400>  3
cgggatccac ctaaaaatcc cgtcaccac                                          29

<210>  4

<211>  23

<212>  DNA

<213>  Artificial Sequence

<220>

<223>  oligonucleotide primer

<400>  4
cgggatccaa cacaactatg ccg                                                23

<210>  5

<211>  27

<212>  DNA

<213>  Artificial Sequence

<220>

<223>  oligonucleotide primer

<400>  5
cgggatcctc cgccaattac tgtggtt                                            27

<210>  6

<211>  19

<212>  DNA

<213>  Artificial Sequence

<220>

<223>  oligonucleotide primer

<400>  6
ttacgactgg tacgttggg                                                     19

<210> 7

<211> 20

<212> DNA

<213> Artificial Sequence

<220>

<223> oligonucleotide primer

<400> 7
gatactcacc taaaatcccg 20

<210> 8

<211> 40

<212> DNA

<213> Artificial Sequence

<220>

<223> oligonucleotide primer

<400> 8
gagaattcac ggccatacca acaggtttta ctggcggaac 40

<210> 9

<211> 18

<212> DNA

<213> Artificial Sequence

<220>

<223> oligonucleotide primer

<400> 9
cttgcgtgca agtggttg 18